# EUROPÄISCHE PATENTANMELDUNG

(11) **EP 2 529 765 A2**
(43) Veröffentlichungstag der Anmeldung: **05.12.2012**
(21) Anmeldenummer: 12167153.1
(22) Anmeldetag: 08.05.2012
(51) Int. Cl.: A61M 1/00

(54) **Wunddrainageverfahren**

(30) Priorität: 31.05.2011 DE 102011076833
(71) Anmelder: ATMOS Medizintechnik GmbH & Co. KG, 79853 Lenzkirch (DE)
(72) Erfinder: Möller, Mario, 79100 Freiburg (DE); Heer, Andreas, 79780 Stühlingen (DE)
(74) Vertreter: Patentanwälte Westphal, Mussgnug & Partner

(57) **Zusammenfassung**

Es wird ein Wunddrainageverfahren beschrieben, bei welchem mittels einer Saugpumpe (10) aus Körperhöhlen oder Wunden von Patienten Sekrete abgesaugt werden, die in einem Sekretauffangbehälter (11) gesammelt werden. Zur intervallweisen Reinigung eines zwischen einem in der Körperhöhle oder Wunde vorgesehenen Katheter (14) angeordneten Saugschlauchs (13) ist ein Spülschlauch (16) vorgesehen, der einerends nahe der Körperhöhle bzw. Wunde mündet und anderenends ein steuerbares Spülventil (17) aufweist. Über eine Regeleinrichtung wird dieses Spülventil (17) von Zeit zu Zeit geöffnet, um mit Hilfe eingebrachter Spülluft den Saugschlauch (13), in dem sich Sekret ablagert, zu reinigen. Zur Messung des Unterdrucks in der Körperhöhle bzw. Wunde ist ein Drucksensor (19) vorgesehen, welcher den Druck in dem zwischen Saugpumpe (10) und Sekretauffangbehälter (11) befindlichen Unterdruckschlauch (12) misst. Dieser Druck entspricht dem Druck in der Körperhöhle bzw. Wunde, wenn die Saugpumpe (10) abgeschaltet ist. Zu diesem Zweck erfolgt die Messung, gesteuert durch eine Regeleinrichtung (18), in den Saugpausen.

## Beschreibung

Die Erfindung betrifft ein Wunddrainageverfahren mittels einer Einrichtung der im Oberbegriff des Anspruchs 1 genannten Art.

Diese Einrichtung besteht im Wesentlichen aus einem Sekretauffangbehälter, einer Saugpumpe, die mit dem Sekretauffangbehälter über einen Unterdruckschlauch verbunden ist, einen in eine Körperhöhle oder Wunde eines Patienten einbringbaren Katheter, der mit dem Sekretauffangbehälter über einen Saugschlauch verbunden ist, einen einerends nahe der Körperhöhle bzw. der Wunde mündenden Spülschlauch, der anderernends von einem Spülventil verschließbar ist, sowie einer Regeleinrichtung, welche die Saugpumpe und das Spülventil unter Berücksichtigung des mit einem Sensor im Unterdruckschlauch gemessenen Druckes steuert.

Eine derartige Einrichtung ist aus WO 2005/061025 bekannt.

Mittels der Saugpumpe werden Körperflüssigkeiten, insbesondere Sekrete, die in der Körperhöhle oder der Wunde des Patienten entstehen, durch Unterdruck abgesaugt und in einem Auffangbehälter gesammelt. Solche Körperflüssigkeiten enthalten in der Regel Proteine, welche bei längerer Verweildauer im Absaugschlauch verhärten und zur Verstopfung oder Verengung des Schlauches führen. Hierdurch wird das weitere Absaugen verschlechtert oder gar unmöglich gemacht. Aus diesem Grunde ist es notwendig oder zumindest zweckmäßig, den Absaugschlauch regelmäßig mit Luft zu spülen, um die eingetretenen Verstopfungen zu beseitigen.

Zu diesem Zweck ist, wie in der oben genannten Druckschrift erläutert, ein Spülschlauch vorgesehen, der einerends in den

Saugschlauch nahe der Körperhöhle bzw. der Wunde mündet und der anderenends von einem Spülventil verschließbar ist. Über das Spülventil kann ein Spülmedium, vorzugsweise atmosphärische Luft, unter der Wirkung der Saugpumpe eingebracht werden.

Bei der bekannten Vorrichtung ist ferner eine Regeleinrichtung vorgesehen, welche die Saugpumpe und das Spülventil unter Berücksichtigung der Druckverhältnisse, nämlich des mit einem Sensor im Unterdruckschlauch gemessenen Druckes, steuert.

Da der im Unterdruckschlauch gemessene Druck wegen der Verstopfungen im Saugschlauch nicht dem Druck in der Körperhöhle bzw. Wunde entspricht, ist zu dessen Messung ein weiterer Sensor erforderlich, welcher den Druck im Spülschlauch ermittelt. Die Messwerte der beiden Sensoren werden in einer Regeleinrichtung gemessen, welche die Saugpumpe und das Spülventil zur exakten Einhaltung des Unterdruckwertes in der Körperhöhle bzw. der Wunde steuert.

Mit der vorliegenden Erfindung wird ein Verfahren für die erläuterte Saugeinrichtung vorgeschlagen, nach welchem mit nur einer einzigen Unterdruckmesseinrichtung, also einem einzigen Sensor, der für die Körperhöhle bzw. Wunde vorgeschriebene Unterdruck präzise gemessen sowie über die Regeleinrichtung eingehalten werden kann.

Gelöst wird diese Aufgabe nach dem Vorschlag gemäß Anspruch 1 dadurch, dass die Saugpumpe intervallweise eingeschaltet wird, dass das Spülventil kurzzeitig zur Ansaugung des gasförmigen Spülmediums bis zum Erreichen eines vorgegebenen Mindestunterdrucks geöffnet wird, wobei der Unterdruck im Unterdruckschlauch ausschließlich in den Saugpausen unmittelbar nach Beendigung des Spülvorganges gemessen wird.

Ist der Saugschlauch freigespült, so entspricht der mit diesem Sensor gemessene Unterdruck dem in der Körperhöhle bzw. der Wunde herrschenden Druck. Durch diese einfache Verfahrensweise kann auf den bei der bekannten Vorrichtung notwendigen zweiten Sensor verzichtet werden.

Die mit der Erfindung vorgeschlagene Maßnahme, das Spülventil nur kurzzeitig bis zur Erreichung eines vorgegebenen Mindestunterdruckes zu öffnen, ist vor allem bei der Sekretabsaugung aus unter Wundverbänden gelegenen Wunden besonders vorteilhaft, um den Unterdruck nicht völlig abzubauen, was das Ablösen des Wundverbandes zur Folge hätte. Um dies optimal zu erreichen, ist die Verwendung eines schnell schaltenden Spülventils empfehlenswert.

Nach dem weiteren Vorschlag gemäß Anspruch 2 sind die Saugintervalle und damit auch die Saugpausen einstellbar. Damit kann die Sekretabsaugung manuell den individuellen Gegebenheiten angepasst werden.

Wie mit Anspruch 3 vorgeschlagen, kann dieser Vorschlag jedoch auch automatisiert werden, wenn nämlich die tatsächliche Sekretfördermenge gemessen wird und die Saugintervalle in Abhängigkeit der Sekretförderleistung der Pumpe gesteuert werden.

Auch lässt sich eine andere Bedarfssteuerung nach dem Vorschlag gemäß Anspruch 4 realisieren, nach welchem die Saugintervallzyklen zeitabhängig, z. B. tageszeitabhängig bzw. lichtabhängig, gesteuert werden. Da die Absaugung nicht geräuschlos erfolgt, ist diese Maßnahme zur Schonung des Patienten während der Nacht- bzw. Ruhezeiten von Vorteil.

Soweit als Spülmedium die atmosphärische Luft verwendet wird, ist das Spülventil mit der Atmosphäre oder, soweit der Spülvorgang intensiviert werden soll, mit einer Druckluftquelle verbindbar. Nach einem weiteren Vorschlag gemäß Anspruch 6 kann das Spülventil auch mit einer Schutzgasquelle verbunden werden, über welche als Spülmedium das Wachstum aerober Bakterien verhinderndes Gas eingeleitet wird. Auch dadurch lässt sich die Häufigkeit der Spülzyklen vermindern.

Dem gleichen Zweck dient der Vorschlag nach Anspruch 7, nach welchem die Schläuche, also die Unterdruck-, Saug- und Spülschläuche, mit einer Heparin-Beschichtung versehen werden, welche die Blutgerinnung in den Schläuchen einschränkt und so die Spülintensität bzw. Spülhäufigkeit minimiert.

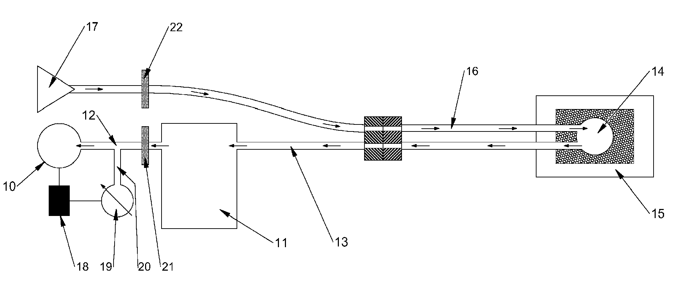

Das erfindungsgemäße Verfahren ist nachstehend anhand einer in der Zeichnung schematisch dargestellten Wunddrainageeinrichtung erläutert.

Diese Wunddrainageeinrichtung besteht im Wesentlichen aus einer Saugpumpe 10, welche über einen Unterdruckschlauch 12 mit einem Sekretauffangbehälter 11 verbunden ist. In den Sekretauffangbehälter 11 mündet ein Saugschlauch 13, an welchen ein in einer von einem Wundverband 15 überdeckten Wunde gelegener Katheter angeschlossen ist. Ferner ist ein Spülschlauch 16 vorgesehen, der nahe des in der Wunde gelegenen Katheters 14 mündet und dessen anderes Ende von einem Spülventil 17 verschlossen ist.

Schließlich ist eine Regeleinrichtung 18 vorgesehen, welche die Saugpumpe 10 sowie das Spülventil 17 steuert. Der im Unterdruckschlauch 12 anstehende Druck wird mittels eines Sensors 19 gemessen. Dieser Messwert wird der Regeleinrichtung 18 zugeführt.

Ein im Unterdruckschlauch 12 angeordnetes Filter 21 verhindert, dass Sekret in die Saugpumpe 10 gelangt. Das im Spülschlauch 16 angeordnete Filter 22 fängt etwaige über das Spülventil 17 eindringende Fremdkörper ab.

Mit einer derartigen Saugeinrichtung erfolgt die Wunddrainage nach folgender Verfahrensweise.

Mittels der Saugpumpe 10 wird ein Unterdruck erzeugt, der bewirkt, dass sich in der Wunde sammelndes Sekret mittels des Saugschlauches 13 und des Katheters 14 in Richtung der eingezeichneten Pfeile abgesaugt wird. Diese Sekrete werden im Sekretauffangbehälter 11 gesammelt. Da die Sekrete Proteine enthalten, die sich bei längerer Verweildauer im Absaugschlauch verhärten und zu Verstopfungen oder Verengungen des Saugschlauches 13 führen, ist eine Reinigung in regelmäßigen Abständen notwendig. Diesem Zwecke dient der Spülschlauch 16, über welchen bei geöffnetem Spülventil 17 Spülluft angesaugt wird, welche in den Saugschlauch 13 gelangt und hier etwaige Ablagerungen beseitigt, welche unter der Wirkung des Unterdrucks in den Sekretauffangbehälter 11 transportiert werden.

Zur Messung des Unterdrucks im System ist ein Sensor 19 vorgesehen, welcher über einen Messschlauch 20 mit dem Unterdruckschlauch 12 verbunden ist. Der im Unterdruckschlauch 12 anstehende Druck entspricht dem Druck innerhalb der vom Wundverband 15 überdeckten Wunde, wenn der Saugschlauch 13 durchgängig, d. h. gereinigt ist. Zur Messung dieses Unterdrucks muss die Saugpumpe 10 abgeschaltet werden, so dass die Messung in den Saugpausen unmittelbar nach Beendigung des Spülvorganges erfolgen kann. Aus diesen Grunde wird nach dem erfindungsgemäßen Vorschlag die Saugpumpe nur intervallweise eingeschaltet, wobei das Spülventil 17 jeweils kurzzeitig zur Ansaugung des gasförmigen Spülmediums bis zum Erreichen eines vorgegebenen Mindestunterdrucks geöffnet wird, worauf der Unterdruck im Unterdruckschlauch in den Saugpausen gemessen wird. Ein minimaler Unterdruck soll im System aufrechterhalten werden, um zu verhindern, dass sich der Wundverband von der Wunde ablöst. Um dies zu erreichen, ist als Luftspülventil ein schnellschaltendes Ventil vorgesehen, das gesteuert von der Regeleinrichtung 18 nur soviel Luft in den Wundverband 15 gelangen lässt, dass der dort befindliche Unterdruck nicht völlig abgebaut wird. Ein Ziel der Erfindung ist, die akustische Belastung des Patienten möglichst gering zu halten, d. h. die geräuschverursachende Saugpumpe nur nach Bedarf einzuschalten. Aus diesem Grunde wird die Saugpumpe nur intervallweise eingeschaltet, wobei die Intervalle in Abhängigkeit von der zeitlich vorher festgestellten Sekretförderleistung bemessen sind. Wenn sich nur wenig Sekret im Schlauch befindet, ist eine Luftspülung nur in größeren Abständen erforderlich.

Auch wird vorgeschlagen, während der Nacht- und Ruhezeiten die Luftspülung seltener zu aktivieren, d. h. die geräuschintensiven Spülintervallzyklen zu verlängern. Zu diesem Zweck ist vorzugsweise ein Lichtsensor vorgesehen, mit welchem tageszeitabhängige Steuersignale für die Steuerung der Regeleinrichtung erzeugt werden. Eine Intervallzyklusverlängerung während der Tages-Ruhezeiten lässt sich mit einer internen Uhr realisieren.

Auch durch die Verwendung von Schutzgas, welches Wachstum von aeroben Bakterien verhindert, zur Spülung des Saugschlauches sowie heparinbeschichteter Sekretschläuche, welche die Blutgerinnung im Schlauch einschränken, lassen sich Spülintensität und Spülhäufigkeit minimieren, was gleichfalls zum schonenden Betrieb der Wunddrainage beiträgt.

### Bezugszeichenliste

- 10: Saugpumpe
- 11: Sekretauffangbehälter
- 12: Unterdruckschlauch
- 13: Saugschlauch
- 14: Katheter
- 15: Wundverband
- 16: Spülschlauch
- 17: Spülventil
- 18: Regeleinrichtung
- 19: Sensor

- 20: Messschlauch
- 21: Filter
- 22: Filter

## Patentansprüche

1. Wunddrainageverfahren mittels einer Einrichtung, bestehend aus einem Sekretauffangbehälter (11), einer Saugpumpe (10), die mit dem Sekretauffangbehälter (11) über einen Unterdruckschlauch (12) verbunden ist, einem in eine Körperhöhle oder Wunde eines Patienten einbringbaren Katheter (14), der mit dem Sekretauffangbehälter (11) über einen Saugschlauch (13) verbunden ist, einem einerends in den Saugschlauch (13) nahe der Körperhöhle bzw. der Wunde mündenden Spülschlauch (16), der anderenends von einem Spülventil (17) verschließbar ist, sowie einer Regeleinrichtung (18), welche die Saugpumpe (10) und das Spülventil (17) unter Berücksichtigung des mit einem Sensor (19) im Unterdruckschlauch (12) gemessenen Druckes steuert, **dadurch gekennzeichnet, dass** die Saugpumpe (10) intervallweise eingeschaltet wird, dass das Spülventil kurzzeitig zur Ansaugung des gasförmigen Spülmediums bis zum Erreichen eines vorgegebenen Mindestunterdrucks geöffnet wird, wobei der Unterdruck im Unterdruckschlauch (12) in den Saugpausen unmittelbar nach Beendigung des Spülvorganges gemessen wird.

2. Wunddrainageverfahren nach Anspruch 1,
**dadurch gekennzeichnet, dass** die Saugintervalle einstellbar sind.

3. Wunddrainageverfahren nach Anspruch 2,
**dadurch gekennzeichnet, dass** die Saugintervalle in Abhängigkeit der gemessenen Sekretfördermenge gesteuert werden.

4. Wunddrainageverfahren nach Anspruch 2,
**dadurch gekennzeichnet, dass** die Saugintervallzyklen zeitabhängig bzw. lichtabhängig gesteuert werden.

5. Wunddrainageverfahren nach einem der Ansprüche 1 bis 4, **dadurch gekennzeichnet, dass** das Spülventil mit der Atmosphäre oder einer Druckluftquelle verbindbar ist.

6. Wunddrainageverfahren nach einem der Ansprüche 1 bis 4, **dadurch gekennzeichnet, dass** das Spülventil mit einer Schutzgasquelle zur Einleitung eines das Wachstum aerober Bakterien verhindernden Gases verbindbar ist.

7. Einrichtung zur Durchführung des Wunddrainageverfahrens nach einem der Ansprüche 1 bis 6,
**dadurch gekennzeichnet, dass** die Unterdruck-, Saug- und Spülschläuche (12, 13 und 16) eine Heparinbeschichtung aufweisen.
